# EUROPEAN PATENT APPLICATION

(11) **EP 4 001 919 A1**
(43) Date of publication of application: **25.05.2022**
(21) Application number: 20382988.2
(22) Date of filing: 13.11.2020
(51) Int. Cl.: G01N 33/564

(54) **PREDICTIVE BIOMARKERS OF AUTOIMMUNITY IN PATIENTS TREATED WITH ALEMTUZUMAB**

(71) Applicant: Fundación para la Investigación Biomédica del Hospital Universitario Ramón y Cajal, 28034 Madrid (ES)
(72) Inventor: VILLAR GUIMERANS, Luisa María, 28034 Madrid (ES); WALO DELGADO, Paulette Esperanza, 28034 Madrid (ES); MEDINA HERAS, Silvia, 28034 Madrid (ES); MONREAL LAGUILLO, Enrique, 28034 Madrid (ES); COSTA-FROSSARD FRANÇA, Lucienne, 28034 Madrid (ES); SAINZ DE LA MAZA CANTERO, Susana, 28034 Madrid (ES)
(74) Representative: Hoffmann Eitle

(57) **Abstract**

The objective of the present invention is determining if a Multiple sclerosis (MS) patient's blood lymphocyte profile before alemtuzumab treatment initiation could identify patients with an increased risk of developing later autoimmunity. We herein describe, for the first time, that the B/4TNF ratio can be a useful tool to stratify the risk of autoimmunity in MS patients who are going to initiate alemtuzumab treatment.

## Description

### Technical field of the invention

The invention relates to a method of predicting a subject's response to alemtuzumab antibody therapy.

### Background of the invention

Multiple sclerosis (MS) is a chronic autoimmune disease of the central nervous system characterized by inflammation, demyelination, and axonal loss. Different molecules have been approved as disease modifying treatment for MS patients, especially those with the relapsing remitting (RR) form. Alemtuzumab is a humanized monoclonal antibody approved for the treatment of RRMS patients. It is administered as two annual courses resulting in prolonged remission periods and proved to be efficacious for patients with highly inflammatory disease [1].

Alemtuzumab targets CD52, a molecule expressed at high levels on the surface of T and B lymphocytes [2]. It causes a selective depletion of these cells via antibody and complement dependent cytotoxicity and apoptosis [3]. T and B-cell repopulation from hematopoietic precursor cells begins within weeks, with a distinctive pattern; B cells undergo faster repopulation, while T cells remain depleted longer [4]. Repopulation associates with an increased number of CD56bright NK cells [2], a relative increase of regulatory T cells and a decrease in pro-inflammatory cytokine production [5,6]. This may help to rebalance the abnormal immune response, and can explain the long duration of clinical effects in the absence of continuous treatment. As a counterpart, frequent adverse events (AEs) associated with alemtuzumab include infusion associated reactions, infections, and mainly AIAEs (Autoimmune Adverse Events) [7,8]. It is noted that AIAEs are herein defined as the appearance of any autoimmune events during follow-up, with special attention paid to thyroid-associated events, immune thrombocytopenia and autoimmune nephropathy.

In a 6-year follow-up study, the most frequent AIAEs were those involving the thyroid gland, observed in about 42% of patients [9]. Other secondary AIAEs initially described included immune thrombocytopenia and nephropathies. In the post-marketing setting, an increasing number of rare but serious adverse events have been reported, including new AIAEs and sporadic cases of cardiovascular diseases. This led to a temporally restriction of the use of alemtuzumab by the safety committees of the European Medicines Agency's and the U.S. Food and Drug Administration, which was finally revoked [10, 11]. However, AIAEs are so frequent that have limited the use of this drug. Therefore, reliable biomarkers assessing patient risk for developing these complications, and hence, guide patient selection for this particular therapy would be of great clinical importance.

In the present invention, we explored if the blood lymphocyte profile before treatment initiation could be associated with the risk of AIAEs in a group of RRMS patients treated with alemtuzumab.

### Summary of the invention

The objective of the present invention is determining if a MS, preferably RRMS, patient's blood lymphocyte profile before alemtuzumab treatment initiation could identify patients with an increased risk of developing later autoimmunity. For this purpose, we have carried out a multicenter prospective longitudinal study including 59 RRMS patients treated with alemtuzumab for a median time of 2.8 years in five Spanish hospitals. Blood samples were collected before initiating treatment with alemtuzumab and different leucocyte subsets determined by flow cytometry. Autoimmune adverse events (AIAEs) were assessed during follow-up. Differences were explored using Man-Whitney U tests. Cut-off values were established using ROC curves. Odds ratios were calculated by Fisher exact tests.

As a result of this study, we have determined that patients developing AIAEs had higher percentages of B cells (p=0.001), being differences mainly due to plasmablasts (p=0.003) and naive cells (p=0.02). By contrast, AIAEs- patients showed a clear increase of the percentages of CD4+ T cells producing TNF-alpha (p=0.019). The ratio between both lymphocyte subsets (B/4TNF) was the best variable to differentiate both groups of patients (p=0.0006). A B/4TNF ratio higher than 0.5 closely associated with higher risk of autoimmune AIAEs (Odds ratio: 52.0, 95% confidence interval: 6.5-565.6, p=2.1x10⁻⁶).

Therefore, the B/4TNF ratio can be a useful tool to stratify the risk of autoimmunity in MS patients who are going to initiate alemtuzumab treatment.

### Brief description of the invention

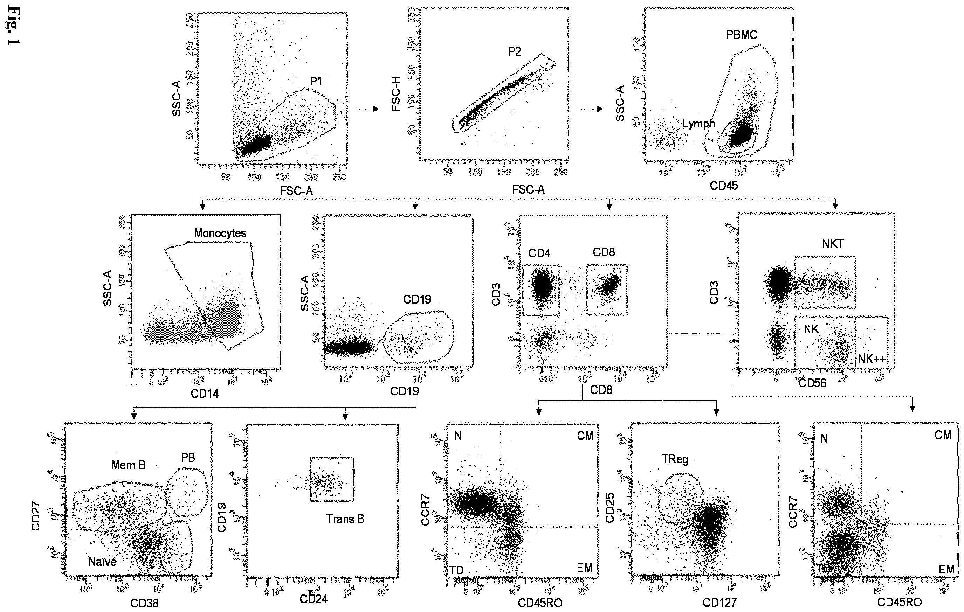

**Figure 1****.** Gating strategy. Peripheral blood mononuclear cells (PBMC) were first gated for excluding debris and apoptotic cells (gate P1) and duplets (gate P2). Lymphocytes were recognized by CD45 staining and low SSC (gate Lymph). Monocytes were recognized as CD14++ cells with intermediate SSC (gate Monocytes). B cells were detected by the expression of CD19 (gate CD19) and subdivided by CD27 staining into memory cells: CD27+ (gate Mem B), naive: CD27- (gate Naive), plasmablasts: CD27hi, CD38hi (gate PB), and transitional B cells: CD27-, CD24+ (gate Trans B). Total CD4+ T cells were identified as CD3+ CD8- T cells (gate T CD4), while CD8+ T cells were gated as CD3+ CD8++ cells (gate T CD8). CD4 and CD8 T cells were classified as naive: CCR7+ CD45RO- (gate N), central memory: CCR7+ CD45RO+ (gate CM), effector memory: CCR7- CD45RO+ (EM), and terminally differentiate: CCR7- CD45RO- (gate TD). CD4+ T regulator cells were identified as CD25hi and CD127-/low (gate T reg). NK cells were identified based on their CD56 expression intensity and CD3 co-expression into CD56+ CD3- (gate NK), CD56+ CD3+ (gate NKT), and CD56++ CD3- (gate NK++). Abbreviation: FSC-A, Forward Scatter-Area; FSC-H, Forward Scatter-Height; SSC, Side Scatter.

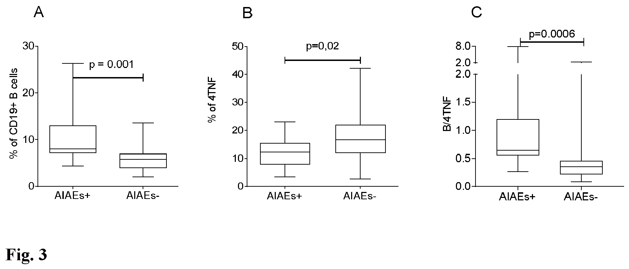

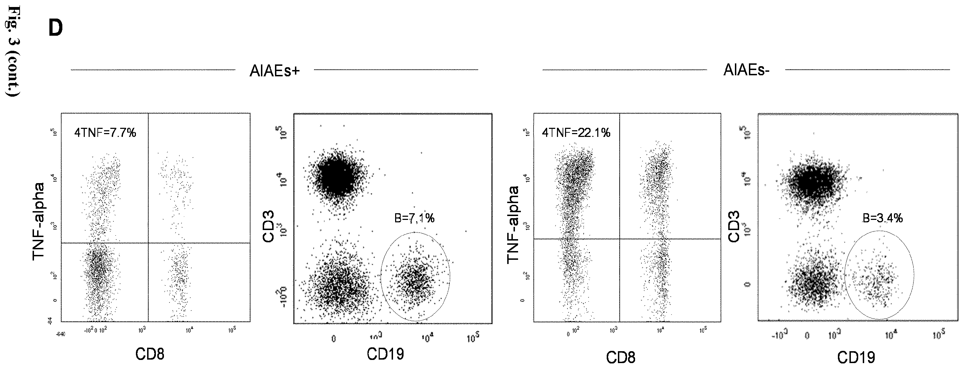

**Figure 3****.** Percentages of cells subsets of alemtuzumab-treated patients classified according to the presence (+, n=14) or absence (-, n=40) of adverse autoimmune events (AIAEs). A) Percentages of total B cells (B). B) Percentages of CD4+ T cells producing TNF-alpha (4TNF). C) B/4TNF ratio. D) Representative dot plots showing B and 4TNF cells. All percentages are relative to total peripheral blood mononuclear cells.

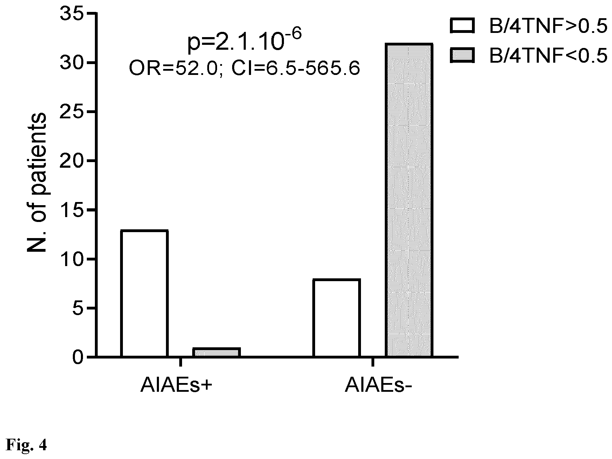

**Figure 4****.** Proportion of patients with (+) or without (-) autoimmune adverse events (AIAES) classified according to B cells / of CD4+ T cells producing TNF-alpha (B/4TNF) ratio. Abbreviations: OR, odds ratio; CI, confidence interval.

### Description of the invention

### Definition

The terms "treatment" and "therapy", as used in the present application, refer to a set of hygienic, pharmacological, surgical and/or physical means used with the intent to cure and/or alleviate a disease and/or symptoms with the goal of remediating the health problem. The terms "treatment" and therapy" include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application.

The terms "individual", "patient" or "subject" are used interchangeably in the present application and are not meant to be limiting in any way. The "individual", "patient" or "subject" can be of any age, sex and physical condition.

The terms "Multiple Sclerosis" or "MS" refer to a chronic inflammatory disease of the central nervous system (CNS) of unknown etiology, in which immune cells migrate to the central nervous system inducing demyelination and axonal damage.

The terms "relapse remitting Multiple Sclerosis" or "RRMS" refer to a type of MS characterized by flare-ups or relapses. The flare-ups or relapses may occur after a period of inactivity of the disease.

The terms "Peripheral blood mononuclear cell" or "PBMC" refer to any peripheral blood cell having a round nucleus.

The terms "Peripheral blood mononuclear cell subset" or "PBMC subset" refer to a specific type of PBMC characterized by expressing a particular surface antigen and/or cytokine.

The term "surface antigen" refers to any membrane-bound target, usually a transmembrane protein, which can be used to characterize a PBMC and categorize the PBMC into a PBMC subset. Examples of surface antigens include: CD4, CD8, CCR7, CD45RO, CD3, CD25, CD127, CD19, CD38, CD24 and CD56.

The term "cytokine" refers to a broad class of small proteins that are important in cell signaling. Examples of cytokines include interleukin-10 (IL-10), interferon-y (IFN-γ), granulocyte-macrophage colony-stimulating factor (GM-CSF), tumor necrosis factor alpha (TNF-α) and interleukin-17 (IL-17).

The term "isolated blood sample" refers to any blood sample which has been previously obtained, i.e. isolated, from a patient. It is to be understood that the act of obtaining the blood samples from a patient is not an aspect of the present invention.

The term "Alemtuzumab" is a recombinant DNA-derived humanized IgG1 kappa monoclonal antibody that is directed against the 21-28 kDa cell surface glycoprotein CD52 (A. Klement (7 January 2014). "Multiple-Sklerose-Behandlung". Österreichische Apothekerzeitung (in German) (1/2014): 24f).

### Description

The search for biomarkers predicting the appearance of new AIAEs after alemtuzumab treatment is of the utmost importance, since these side effects limit the use of this drug, which has proven to be very efficacious in patients with aggressive MS [1].

The data obtained in the pivotal phase 3 trials suggested that the pattern of T- and B-cell depletion and repopulation following alemtuzumab treatment could influence the appearance of the secondary autoimmunity [14]. However, repopulation kinetics of the peripheral lymphocyte subsets, measured as absolute cell counts, are comparable between patients with or without AIAEs [15].

On the other hand, high serum levels of IL-21, a cytokine promoting T and B-cell differentiation and antibody production [16], were proposed as biomarkers to predict secondary autoimmunity after alemtuzumab treatment [17]. However ulterior analyses limited its use as a biomarker since there is some overlapping between patients who were going to develop AAIEs and those who not, and detection tests currently available could not distinguish accurately between both groups of patients [19]. Also pre-treatment presence of serum anti-thyroid autoantibodies associated with increased risk for clinical onset of thyroid autoimmunity after alemtuzumab treatment [20], however this biomarker would only benefit a small group of patients [21].

On the other hand, for the present invention, we focused on studying the immune cell profile before alemtuzumab treatment, assessing both the absolute counts and percentages of the peripheral blood mononuclear cells. We did not find differences in the absolute counts of any cell subset, as previously reported by other groups [15]. However, patients who later develop autoimmunity presented with higher percentages of B cells, particularly of the naive and plasmablast subsets. By contrast, patients who did not develop AIAEs showed a clear increase in the percentage of CD4+ T cells producing TNF-alpha. An increased percentage of naive B lymphocytes suggests a high turnover rate of B cells from hematopoietic precursors. In addition, plasmablasts are effector cells that produce immunoglobulins at a high rate, playing an important role in inflammation in MS [22]. CD4+ T cells producing TNF alpha drive TH1 responses, which play an important role in MS pathogenesis [24]. MS is a heterogeneous disease with different immunological mechanisms playing a main role in the inflammatory response (25). Our data strongly suggest that the immunological mechanisms predominating in individual patients not only influence the response to treatment in MS (26) but can have a clear influence in the side effects. In alemtuzumab treated patients, B cell repopulation is completed about six months after administrating the drug, while that of CD4+ T cells may last up to two years [6]. In this context, a high number of plasmablasts may concur with low numbers of regulatory cells for prolonged periods of time, thus increasing the probability of other autoimmune diseases based on B cells, as those mainly occurring after alemtuzumab treatment. When we explored B lymphocytes and CD4+ T cells producing TNF alpha in 41 patients after a year of the first alemtuzumab cycle, we observed that those with high B/4TNF ratios at baseline maintained high percentages of plasmablasts after a year. This data supports the association of the immunological profile with plasmablasts as the predominant mechanism driving the autoimmune response with higher risk of AIAEs after alemtuzumab treatment.

In line with these findings we observed that baseline B/4TNF ratio accurately predicted the risk of secondary autoimmunity. Thirteen out of 14 patients having AIAEs showed a pre-treatment ratio upper than 0.5, whilst only eight of 40 patients lacking secondary autoimmunity showed these high B/4TNF values and the possibility of some of them driving autoimmunity during follow-up cannot be ruled out since AIAEs can appear after years of alemtuzumab treatment. These results strongly suggest that the B/4TNF ratio can be an important tool to identify MS patients at low risk of autoimmunity if treated with alemtuzumab.

The present invention thus provides, in a first aspect, a method for monitoring or predicting the risk of Autoimmune adverse events (AIAEs) derived from the treatment with alemtuzumab in a patient who suffers from Multiple Sclerosis, comprising the steps of: (a) isolating peripheral blood mononuclear cells from isolated blood samples obtained before and optionally after alemtuzumab treatment; and (b) quantifying the amount of CD 19 B lymphocytes and CD4+ T cells producing TNF alpha in the one or more isolated peripheral blood samples and obtaining the ratio B/4TNF (CD19 B lymphocytes/CD4+ T cells producing TNF alpha), and (c) identifying the patient as having: (i) a low risk of autoimmunity if treated with alemtuzumab when the ratio B/4TNF (CD19 B lymphocytes/CD4+ T cells producing TNF alpha) is reduced compared to a reference score or value; and identifying the patient as having: (ii) a high risk of autoimmunity if treated with alemtuzumab when the ratio B/4TNF (CD19 B lymphocytes/CD4+ T cells producing TNF alpha) is increased compared to a reference value or score.

In a preferred embodiment of the first aspect of the invention, the amount of CD19 B lymphocytes used to calculate the ratio are selected from the group consisting of the total amount of CD19 B lymphocytes or of the amount of the naive and plasmablast subsets.

In still another preferred embodiment of the first aspect of the invention, the reference value or score is a cutoff as determined by a receiver operating characteristic (ROC) curve; wherein the ROC curve is based on the distribution of patients having Autoimmune adverse events (AIAEs) in response to treatment with alemtuzumab and patients not having Autoimmune adverse events (AIAEs) in response to treatment with alemtuzumab. Preferably, the cut-off value is of about 0.50 to identify patients at risk of AIAEs.

It is thus a goal of the first aspect of the invention to predict the risk of Autoimmune adverse events (AIAEs) derived from the treatment with alemtuzumab in a patient who suffers from Multiple Sclerosis. To achieve this, a sample of blood is usually taken from the patient before the treatment begins. The PBMCs are then isolated from the sample, and the amount of cells in the specified PBMC subsets are quantified and used to elaborate the ratio B/4TNF. The present invention shows that one could use this ratio to predict whether the treatment will cause secondary autoimmunity. I particular, the present invention shows that one could use this ratio to predict whether the treatment will cause the appearance of any autoimmune events during follow-up, with special attention paid to thyroid-associated events, immune thrombocytopenia and autoimmune nephropathy. It is also envisioned that the method of the present invention could be used to continuously monitor the risk of Autoimmune adverse events (AIAEs) derived from the treatment with alemtuzumab in a patient who suffers from Multiple Sclerosis.

The present invention further provides, in a second aspect, a method of predicting a treatment outcome with alemtuzumab in a patient who suffers from Multiple Sclerosis, comprising the steps of:
a) quantifying the amount of CD19 B lymphocytes and CD4+ T cells producing TNF alpha in one or more isolated peripheral blood samples obtained from the patient, and obtaining an assessment score from the value/s obtained from the ratio B/4TNF (CD19 B lymphocytes/CD4+ T cells producing TNF alpha);
   and
b) comparing the value of said assessment score of step a) with a reference value or reference score,
wherein said corresponding reference (value or score) is linked to a treatment outcome in patients having been treated with alemtuzumab, and
wherein the deviation between the assessment score of step a) with the score or value of reference derived from the comparison step b) is used to predict a treatment outcome for said patient with Multiple Sclerosis when treated with alemtuzumab.

In a preferred embodiment of the second aspect of the invention, the amount of CD 19 B lymphocytes used to calculate the ratio are selected from the group consisting of the total amount of CD19 B lymphocytes or of the amount of the naive and plasmablast subsets.

In still another preferred embodiment of the second aspect of the invention, the reference value or score is a cutoff as determined by a receiver operating characteristic (ROC) curve; wherein the ROC curve is based on the distribution of patients having Autoimmune adverse events (AIAEs) in response to treatment with alemtuzumab and patients not having Autoimmune adverse events (AIAEs) in response to treatment with alemtuzumab.

In another preferred embodiment of the second aspect of the invention, the cut-off value is of about 0.50 to identify patients at risk of AIAEs.

The present invention further provides, in a third aspect, a composition comprising alemtuzumab for use in treating Multiple Sclerosis in a group of patients characterized by having a ratio B/4TNF (CD19 B lymphocytes/CD4+ T cells producing TNF alpha) in one or more isolated peripheral blood samples obtained from the patient lower than a reference level, wherein the reference level is a cutoff as determined by a receiver operating characteristic (ROC) curve; wherein the ROC curve is based on the distribution of patients having Autoimmune adverse events (AIAEs) in response to treatment with alemtuzumab and patients not having Autoimmune adverse events (AIAEs) in response to treatment with alemtuzumab.

The present invention further provides, in an alternative third aspect, a composition comprising alemtuzumab for use in treating Multiple Sclerosis in a group of patients identified in accordance with the method of the first aspect as having a low risk of autoimmunity if treated with alemtuzumab.

In a preferred embodiment of the third aspect of the invention, the amount of CD19 B lymphocytes used to calculate the ratio are selected from the group consisting of the total amount of CD19 B lymphocytes or of the amount of the naive and plasmablast subsets.

In another preferred embodiment of the third aspect of the invention, the cut-off value is of about 0.50 to identify patients at risk of secondary autoimmunity.

In a preferred embodiment of any of aspects one to three, MS is RRMS.

In another preferred embodiment of any of aspects one to three, the amount of CD19 B lymphocytes and CD4+ T cells producing TNF alpha in the one or more isolated peripheral blood samples are quantified by flow cytometry. In a preferred embodiment, the amount of CD19 B lymphocytes and CD4+ T cells producing TNF alpha in the one or more isolated peripheral blood samples is quantified using flow cytometry. Preferably, the isolated peripheral blood mononuclear cells are stained using antibodies recognizing surface antigens and then quantified using flow cytometry. More preferably, the isolated peripheral blood mononuclear cells are stained using antibodies recognizing surface antigens, permeabilized, then stained intracellularly with antibodies recognizing the CD19 B lymphocytes and the CD4+ T cells producing TNF alpha and then quantified using flow cytometry.

The present invention further provides, in a fourth aspect, a kit comprising antibodies for identifying the ratio B/4TNF (CD19 B lymphocytes/CD4+ T cells producing TNF alpha). In an alternative embodiment, the kit which comprises antibodies used to identify the ratio B/4TNF (CD19 B lymphocytes/CD4+ T cells producing TNF alpha) comprises: (i) a tube or recipient comprising monoclonal antibodies use to identify the ratio B/4TNF (CD19 B lymphocytes/CD4+ T cells producing TNF alpha), or several tubes or recipients comprising separately or in combination each of these antibodies, and (ii) optionally a washing or buffer solution. Preferably, the antibodies are labeled. It is noted that, optionally, the kit may further comprise any reagents (buffers, solutions, dyes...) needed to implement the method of any of the first or second aspects of the invention such as for example, reagents for sample washing, lysing and staining.

Any one of the embodiments of the kit may further comprise alemtuzumab. Preferably, a separate tube or recipient comprising alemtuzumab.

It is particularly noted that different markers can be used to label the antibodies. However, preferred markers are PE (phycoerythrin), Cy7 (Cyanine 7), Cy5.5 (Cyanine 5.5), APC (allophycocyanin), H7, FITC (fluorescein isothiocyanate), BV421 (Brilliant Violet 421), V500 (BD Horizon V500), PercP and conjugates thereof. The conjugates may be PE-Cy5.5, PerCP-Cy5.5, PE-Cy7, and/or APC-H7.

The cells may be fixed and permeabilized with any method and reagent known in the art. For example, the cells may be permeabilized using a Cytofix/Cytoperm Kit (BD Biosciences).

The kits disclosed herein are based on the predictive power of the methods of the present invention. Reference values indicative of a responsive patient can be established using the method disclosed in the present application and the data disclosed in the Examples. In a preferred embodiment, a reference value which is indicative of a non-responsive patient and/or a reference value which is indicative for a responsive patient may be provided with the kit. With the help of the kit, the quantity and/or percentages of each of the cell types can be calculated.

Further, it is to be interpreted that any embodiment of the kit can be used to implement any of the methods of the present invention. Thus, subject matter related to the kit is also related to the methods of the present invention.

Thus, in a further aspect, the kit according to any one of the aforementioned kit embodiments may be used in any of the methods described herein.

The following examples merely illustrate the present invention.

### Examples

### MATERIALS AND METHODS

### Study design

This was a multicenter prospective longitudinal study including 59 patients diagnosed of RRMS according to McDonald criteria [12] who were initiating treatment with alemtuzumab in five Spanish hospitals. Four patients were excluded for presenting autoimmune pathology before treatment initiation, and other one was lost during follow-up. The study was approved by the ethics committee of the Hospital Universitario Ramón y Cajal. All patients signed a written informed consent form before inclusion.

### Patients

Patients were followed for 2.8 [2.5-3.4] years (median [IQ range]) after alemtuzumab treatment initiation. All patients received two courses of alemtuzumab (12 mg/d IV on five consecutive days at baseline and 12 mg/d IV on three consecutive days 12 month later). Two patients received an additional course for presenting new clinical relapses after the second course. Clinical and demographic data of patients at study inclusion are described in Table 1 (as reflected below).

**Table 1. Clinical and demographic features of patients (n=54)**

| Variable | Patients (n=54) |
|---|---|
| Sex (F/M). | 34/20 |
| Age at disease onset [years] - median (25-75% IQR). | 28 (23-34) |
| Age at treatment onset [years] - median (25-75% IQR). | 35 (30-43.8) |
| Disease duration [years] - median (25-75% IQR). | 6 (1-10) |
| EDSS score at treatment onset - median (25-75% IQR). | 3 (1.5-4) |
| Relapse rate in the two previous years - median (25-75% IQR) | 0.83 (0.63-1.7) |
| Previous disease modifying treatments (Yes/No) | 43/11 |
| Number of previous treatments - median (range) | 2 (0-9) |
| Last treatment (Number of patients) | |
| None | 13 |
| IFN beta/Glatiramer acetate/ Teriflunomide | 6 |
| Dimethyl fumarate | 12 |
| Fingolimod | 11 |
| Natalizumab | 12 |
| Time of follow-up [years] - median (25-75% IQR). | 2.8 (2.5-3.4) |
| Alemtuzumab curses (Number of patients) | |
| Two courses | 52 |
| Three courses | 2 |

| | |
|---|---|
| Footnote: n, number of patients; F, Female; M, male; EDSS, Expanded Disability Status Scale; IQR, interquartile range. | |

### Patient follow-up

Patients were evaluated every 3 months to assess the appearance of new AIAEs, the occurrence of new relapses and the Expanded Disability Status Scale (EDSS) score. MRI scans were performed yearly after treatment initiation. Analytical tests, including hemogram, serum analyses of renal and thyroid functions were performed every 3 months. The presence of anti-glomerular basement membrane and anti-thyroid antibodies (including anti-thyroid peroxidase, anti-thyroglobulin and anti-thyroid-stimulating hormone receptor) was assessed every six months.

AIAEs were defined as the appearance of any autoimmune events during follow-up, with special attention paid to thyroid-associated events, immune thrombocytopenia and autoimmune nephropathy.

Response to treatment was also assessed by monitoring the EDSS score, the number of new relapses at every visit and the onset of new lesions on annual MRI scans.

### Flow cytometry analyses

Blood samples were collected before initiating treatment with alemtuzumab. Peripheral blood mononuclear cells (PBMCs) were obtained from heparinized whole blood (20 mL) by Ficoll density gradient centrifugation (Fresenius Kabi) and cryopreserved in aliquots of 5x10⁶ cells until studied. In 41 cases an additional sample was obtained after a year of the first course of alemtuzumab, before administering the second one.

### Monoclonal antibodies

Leukocyte subpopulations where assessed using the following monoclonal antibodies: CD45-V500, CD3-PerCP, CD3-BV421, CD4-APC-H7, CD8-FITC, CD8-APC-H7, CD56APC, CD197-PE, CD45RO-APC, CD25-PE-Cy7, CD127-BV421, CD19-PE-Cy7, CD20-FITC, CD24-FITC, CD27-PE, CD38-APC-H7 and CD14-APC-H7. Intracellular cytokines were stained using the next panel: IL10-PE, IFNγ-FITC, GMCSF-PE, TNFα-PerCP-Cy5.5 and IL17-APC. All monoclonal antibodies were purchased from BD Biosciences, except IL17-APC, from R&D Systems.

### Flow cytometry analyses

To study surface antigens, cells were stained with the respective monoclonal antibodies during 30 min at 4°C in the dark, washed with saline and analyzed in a FACSCanto II flow cytometer (BD Biosciences), as described before [13].

For intracytoplasmic cytokine detection, cells were stimulated with phorbol-12-myristate-13-acetate (PMA, Sigma-Aldrich) and Ionomycin (Sigma-Aldrich, USA), in presence of Brefeldin A (GolgiPlug, BD Biosciences) and Monensin (GolgiStop, BD Biosciences), and then incubated 4 h at 37 °C in 5% CO2 atmosphere. For the analysis of IL-10 producing B cells, PBMC were incubated prior to stimulation with CpG oligonucleotide (InvivoGen, USA) for 20 h. After incubation, surface markers were stained, then the cellular membrane permeabilized with Cytofix/Cytoperm Kit (BD Biosciences), and incubated with intracellular antibodies, following the same protocol previously described [26].

Cells were analyzed using FACSDiva Software V.8.0 (BD Biosciences). A minimum amount of 5x10⁴ events were acquired. A gate including leucocytes and excluding debris and apoptotic cells was established. Gating strategy is defined in Figure 1.

### Statistical analysis

Data were analyzed using the Prism 8.0 statistical package (GraphPad Software, USA). Mann-Whitney U tests was used to explore continuous variables. We used ROC curves to establish cut-off values and Fisher tests to analyze categorical variables. P values below 0.05 were considered significant.

### RESULTS

Fifty-four RRMS patients initiating alemtuzumab treatment were studied. Fourteen (25.9%) experienced AIAEs during follow-up, being all of them autoimmune thyroid alterations. No other autoimmune pathologies secondary to alemtuzumab treatment were observed. No significant differences were found in clinical and demographic characteristics at baseline or during follow-up between patients showing AIAEs (Table 2).

**Table 2: Clinical and demographic characteristics of patients classified according to the appearance of autoimmune adverse events (n=54).**

| Variable | AIAEs (n=14) | No AIAEs (n=40) | p value |
|---|---|---|---|
| Sex (F/M). | 11/3 | 23/17 | |
| Age at disease onset [years] - median (25-75% IQR). | 27 (22-32.5) | 28 (24-36) | ns |
| Age at treatment onset [years] - median (25-75% IQR) | 33 (21-37.5) | 37 (31-46) | ns |
| Disease duration [years] - median (25-75% IQR) | 4 (0.5-8.5) | 6 (3-11) | ns |
| EDSS score at treatment onset - median (25-75% IQR). | 2 (1.5-4) | 3 (1.5-4) | ns |
| Relapse rate in the two previous years - median (25-75% IQR) | 1,2 (0.6-4.9) | 0.8 (0.6-1.3) | ns |
| Prior treatment (Number of patients) | | | ns |
| None (n=13) | 4 | 9 | |
| First line treatments (n=18) | 3 | 15 | |
| Second line treatments (n=23) | 7 | 16 | |

| | | | |
|---|---|---|---|
| Footnote: n, number of patients; AIAEs, Autoimmune adverse events; F, Female; M, male; IQR, interquartile range; ns, not significant; EDSS, Expanded Disability Status Scale; NEDA, No Evidence of Disease Activity. First line treatments: IFN beta, Glatiramer acetate, Teriflunomide, Dimethyl fumarate; Second line treatments: Fingolimod, Natalizumab. | | | |

### Flow cytometry analyses

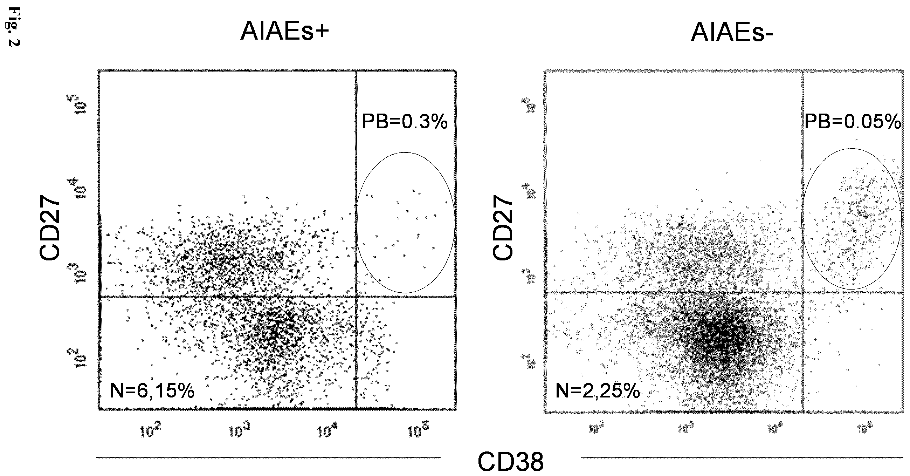

We studied basal lymphocyte profiles. Results of the different leucocytes subsets are shown in Table 3. No significant differences were observed in T lymphocytes, NK cells or monocytes between patients who were going to develop AIAEs or not. However, we found clear differences in the B cell profile. Patients developing AIAEs had higher percentages of B cells (p=0.001), being differences mainly due to plasmablasts (p=0.003) and naive cells (p=0.02) with a trend also observed for memory cells (p=0.05). (Table 3, Figure 2). These differences were not observed in the absolute B cell counts (Table 3).

**Table 3. Baseline peripheral blood mononuclear cells.**

| | AIAEs, n=14 Median (IQ range) | No AIAEs, n=40 Median (IQ range) | p value |
|---|---|---|---|
| B cells | 7.95 (7.07-13.06) | 5.85 (3.97-7.00) | 0.001 |
| Transitional | 0.11 (0.02-0.21) | 0.05 (0.03-0.10) | ns |
| Naive | 4.10 (3.23-7.44) | 2.59 (1.54-4.04) | 0.02 |
| Memory | 2.37 (1.40-4.51) | 1.65 (1.19-2.40) | 0.05 |
| Plasmablasts | 0.14 (0.09-0.25) | 0.09 (0.05-0.11) | 0.003 |
| CD4+ T cells | 32.38 (27.2-42.7) | 38.5 (29.9-45.2) | ns |
| Regulatory | 1.88 (1.63 - 3.34) | 2.23 (1.51-3.39) | ns |
| Naive | 18.5 (13.3-27.9) | 18.1 (9.3-26.3) | ns |
| CM | 8.56 (6.7-9.7) | 9.36 (7.56-12.40) | ns |
| EM | 3.83 (3.03-6.01) | 6.88 (3.40-10.26) | ns |
| TD | 1.09 (0.61-1.62) | 1.26 (0.91-2.08) | ns |
| CD8+ T cells | 10.4 (8.14-15.5) | 9.94 (6.74-15.9) | ns |
| Naïve | 5.25 (3.58-7.32) | 3.10 (1.82-6.52) | ns |
| Central memory | 0.49 (0.35-0.64) | 0.36 (0.23-0.76) | ns |
| Effector memory | 2.22 (1.64-3.95) | 2.06 (1.23-3.10) | ns |
| Terminally differentiated | 2.64 (1.45-3.42) | 2.31 (0.98-6.38) | ns |
| % Natural killer cells | 17.3 (11.6-21.2) | 18.9 (13.8-24.7) | ns |
| CD56^{dim} | 17.3 (11.6-21.2) | 14.1 (10.1-18.0) | ns |
| CD56^{bright} | 0.87 (0.73-1.27) | 0.99 (0.56-1.17) | ns |
| NKT | 2.77 (1.58-4.08) | 3.24 (1.80-5.76) | ns |
| % Monocytes | 13.3 (6.08-21.2) | 12.20 (8.68-20.6) | ns |

| | | | |
|---|---|---|---|
| Footnote: AIAEs, Autoimmune adverse events; IQ, Interquartile; ns, not significant. The percentages were calculated over total peripheral blood mononuclear cells. | | | |

When exploring intracellular cytokine production (Table 4), we found a clear increase of the percentages of CD4+ T cells producing TNF-alpha in patients who did not develop AIAEs during follow-up (p=0.019). No other significant find differences in B or T cells producing pro- or anti-inflammatory cytokines were found.

**Table 4. Percentages of cytokine producing lymphocytes.**

| | AIAEs, n=14 Median (IQ range) | No AIAEs, n=40 Median (IQ range) | p value |
|---|---|---|---|
| IL10+ B cells | 11.7 (7.74-19.6) | 8.15 (6.15-12.1) | ns |
| TNFa+ B cells | 1.69 (1.11-2.98) | 2.40 (1.30-5.04) | ns |
| GMCSF+ B cells | 0.51 (0.27-0.64) | 0.30 (0.20-0.37) | ns |
| IL10+ CD4+ T cells | 0.18 (0.10-0.30) | 0.14 (0.09-0.20) | ns |
| TNFa+ CD4+ T cells | 12.2 (7.91-15.6) | 16.7 (11.9-22.0) | 0.019 |
| GMCSF+ CD4+ T cells | 1.27 (0.86-1.66) | 1.36 (1.00-2.68) | ns |
| IFNg+ CD4+ T cells | 2.54 (2.08-4.50) | 3.90 (2.30-5.69) | ns |
| IL17+ CD4+ T cells | 0.12 (0.08-0.26) | 0.13 (0.09-0.24) | ns |
| IL10+ CD8+ T cells | 0.11 (0.04-0.20) | 0.11 (0.05-0.27) | ns |
| TNFa+ CD8+ T cells | 3.06 (1.98-5.62) | 5.34 (3.00-8.00) | 0.05 |
| GMCSF+ CD8+ T cells | 0.65 (0.38-1.12) | 0.79 (0.50-1.16) | ns |
| IFNg+ CD8+ T cells | 2.44 (1..47-3.88) | 3.10 (1.76-4.88) | ns |
| IL17+ CD8+ T cells | 0.06 (0.03-0.14) | 0.09 (0.05-0.10) | ns |

| | | | |
|---|---|---|---|
| Footnote: AIAEs, Autoimmune adverse events; IQ, Interquartile; ns, not significant. The percentages were calculated over total peripheral blood mononuclear cells. | | | |

Since AIAEs+ patients showed high B cell percentages and low proportion of CD4+ TNF-alpha+ T cells and AIAEs- ones had the opposite pattern, we explored if the ratio between both variables (B/4TNF) could predict the occurrence of autoimmunity. Patients who developed autoimmunity during follow-up showed increased values of this ratio (p=0.0006, figure 4). Using a ROC curve analysis (area under the curve = 0.8, p=0.0009) we established a cut-off value of 0.50 to identify patients at risk of secondary autoimmunity. Thirteen out of 14 patients presenting AIAEs had a B/4TNF ratio lower than 0.5, while only eight of the 40 patients who did not present AIAEs during follow-up had a B/4TNF ratio higher than 0.5 (Odd ratio =52.0, confidence interval: 6.5-565.6, p=2.1x10⁻⁶ (Figure 4).

To explore if changes observed at baseline remained after a year of alemtuzumab treatment, we analyzed lymphocyte subsets giving significant results at baseline in peripheral blood mononuclear samples of 41 patients (14 showing B/4TNF ratio>0.5 and 26 with values>0.5). The samples were obtained before administering the second cycle of the drug. Results are shown in table 5. The only subset maintaining significant differences was plasmablasts that remained high a year after administering the first alemtuzumab cycle.

### REFERENCES

[1] Cohen JA, Coles AJ, Arnold DL, et. al.: Alemtuzumab versus interferon beta 1a as first-line treatment for patients with relapsing-remitting multiple sclerosis: a randomised controlled phase 3 trial. Lancet 2012; 380: pp. 1819-1828
[2] Gross CC, Ahmetspahic D, Ruck T, et al. Alemtuzumab treatment alters circulating innate immune cells in multiple sclerosis. Neurol Neuroimmunol Neuroinflamm. 2016;3(6):e289. Published 2016 Oct 12.
[3] Buonomo AR, Zappulo E, Viceconte G, Scotto R, Borgia G, Gentile I. Risk of opportunistic infections in patients treated with alemtuzumab for multiple sclerosis. Expert Opin Drug Saf. 2018;17(7):709-717.
[4] Thompson, S.A. et al. B-cell reconstitution and BAFF after alemtuzumab (Campath 1H) treatment of multiple sclerosis. J. Clin. Immunol. 2010, 30, 99-105.
[5] De Mercanti S et al (2016). Alemtuzumab long-term immunologic efect: Treg suppressor function increases up to 24 months. Neurol Neuroimmunol Neuroinfamm 3:e194.
[6] Zhang X et al (2013). Diferential reconstitution of T cell subsets following immunodepleting treatment with alemtuzumab (anti-CD52 monoclonal antibody) in patients with relapsing-remitting multiple sclerosis. J Immunol 191:5867-5874.
[7] Ziemssen T, Thomas K (2017). Alemtuzumab in the long-term treatment of relapsing-remitting multiple sclerosis: an update on the clinical trial evidence and data from the real world. Ther Adv Neurol Disord 10:343-359.
[8] Tuohy O, Costelloe L, Hill-Cawthorne G, et al. Alemtuzumab treatment of multiple sclerosis: long term safety and efficacy. J neurol Neurosurg Physiatry. 2015;86(2):208-215.
[9] Dayan C et al (2016). Autoimmunity in patients treated with alemtuzumab for relapsing-remitting multiple sclerosis: 6-year follow-up of the CARE-MS studies. Mult Scler 22:168.
[10] https://www.ema.europa.eu/en/medicines/human/referrals/lemtrada
[11] https://www.fda.gov/drugs/drug-safety-and-availability/fda-warns-about-rare-serious-risks-stroke-and-blood-vessel-wall-tears-multiple-sclerosis-drug
[12] Thompson AJ, Banwell BL, Barkhof F, et al. Diagnosis of multiple sclerosis: 2017 revisions of the McDonald criteria. Lancet Neurol. 2018;17(2):162-173.
[13] Medina S, Villarrubia N, Sainz de la Maza S, et al. Optimal response to dimethyl fumarate associates in MS with a shift from an inflammatory to a tolerogenic blood cell profile. Mult Scler J 2018;24:1317-1327.
[14] Baker D, Herrod SS, Alvarez-Gonzalez C, Giovannoni G, Schmierer K. Interpreting Lymphocyte Reconstitution Data From the Pivotal Phase 3 Trials of Alemtuzumab. JAMA Neurol. 2017;74(8):961-969.
[15] Heinz Wiendl, MD, PhD, Matthew Carraro, MD. Lymphocyte pharmacodynamics are not associated with autoimmunity or efficacy after alemtuzumab. Neurol Neuroimmunol Neuroinflamm 2020;7:e635.
[16] Costelloe L, Jones J, Coles A. Secondary autoimmune diseases following alemtuzumab therapy for multiple sclerosis. Expert Rev Neurother. 2012 Mar;12(3):335-41. doi: 10.1586/ern.12.5. PMID: 22364332.
[17] Jones JL, Phuah CL, Cox AL, et al. IL-21 drives secondary autoimmunity in patients with multiple sclerosis, following therapeutic lymphocyte depletion with alemtuzumab (Campath-1H). J Clin Invest. 2009;119(7):2052-2061.
[18] Long D, Chen Y, Wu H, Zhao M, Lu Q. Clinical significance and immunobiology of IL-21 in autoimmunity [published correction appears in J Autoimmun. 2020 Jul;111:102455]. J Autoimmun. 2019;99:1-14.
[19] Azzopardi L, Thompson SA, Harding KE, et al. Predicting autoimmunity after alemtuzumab treatment of multiple sclerosis. J Neurol Neurosurg Psychiatry. 2014;85(7):795-798.
[18] Reindl M. Anti-thyroid autoantibodies as biomarkers for alemtuzumab associated thyroid autoimmunity. EBioMedicine. 2019 Sep;47:22-23. doi: 10.1016/j.ebiom.2019.08.065. Epub 2019 Aug 31. PMID: 31481325; PMCID: PMC6796534.
[21]. Marcus Riendl. Anti-thyroid autoantibodies as biomarkers for alemtuzumab associated thyroid autoimmunity. COMMENTARY| VOLUME 47, P22-23, SEPTEMBER 01. 2019, https://www.thelancet.com/journals/ebiom/article/PIIS2352-3964(19)30587-0/fulltext#secst0015
[22] Cepok S, Rosche B, Grummel V, Vogel F, Zhou D, Sayn J, Sommer N, Hartung HP, Hemmer B. Short-lived plasma blasts are the main B cell effector subset during the course of multiple sclerosis. Brain. 2005 Jul;128(Pt 7):1667-76. doi: 10.1093/brain/awh486. Epub 2005 Mar 30. PMID: 15800022.
[23] Baker D, Ali L, Saxena G, Pryce G, Jones M, Schmierer K, Giovannoni G, Gnanapavan S, Munger KC, Samkoff L, Goodman A and Kang AS (2020) The Irony of Humanization: Alemtuzumab, the First, But One of the Most Immunogenic, Humanized Monoclonal Antibodies. Front. Immunol. 11:124.
[24] Chitnis T. The role of CD4 T cells in the pathogenesis of multiple sclerosis. Int Rev Neurobiol. 2007;79:43-72.
[25] Kuhlmann T, Ludwin S, Prat A, Antel J, Brück W, Lassmann H. An updated histological classification system for multiple sclerosis lesions. Acta Neuropathol. 2017;133(1):13-24.
26. Alenda R, Costa-Frossard L, Alvarez-Lafuente R, Espejo C, Rodriguez-Martin E, de la Maza SS, et al. Blood lymphocyte subsets identify optimal responders to IFN-beta in MS. J Neurol. 2018 Jan;265(1):24-31.

## Claims

1. A method for predicting the risk of Autoimmune adverse events (AIAEs) derived from the treatment with alemtuzumab in a patient who suffers from Multiple Sclerosis, comprising the steps of: a) obtaining the ratio B/4TNF (CD19 B lymphocytes / CD4+ T cells producing TNF alpha) from the amount of CD19 B lymphocytes and CD4+ T cells producing TNF alpha obtained from one or more isolated peripheral blood samples isolated from the patient, and identifying the patient as having: (i) a low risk of autoimmunity if treated with alemtuzumab when the ratio B/4TNF (CD19 B lymphocytes/CD4+ T cells producing TNF alpha) is reduced compared to a reference score or value; and identifying the patient as having: (ii) a high risk of autoimmunity if treated with alemtuzumab when the ratio B/4TNF (CD19 B lymphocytes/CD4+ T cells producing TNF alpha) is increased compared to a reference value or score.

2. The method of claim 1, wherein the amount of CD19 B lymphocytes used to calculate the ratio are selected from the group consisting of the total amount of CD19 B lymphocytes or of the amount of the naive and plasmablast subsets.

3. The method of any of claims 1 or 2, wherein the reference value or score is a cutoff as determined by a receiver operating characteristic (ROC) curve; wherein the ROC curve is based on the distribution of patients having Autoimmune adverse events (AIAEs) in response to treatment with alemtuzumab and patients not having Autoimmune adverse events (AIAEs) in response to treatment with alemtuzumab.

4. The method according to claim 3, wherein the cut-off value is of about 0.50 to identify patients at risk of AIAEs.

5. The method of any of claims 1 to 4, wherein the method is implemented by a computer.

6. A method of predicting a treatment outcome with alemtuzumab in a patient who suffers from Multiple Sclerosis, comprising the steps of:
a) obtaining an assessment score from the value/s obtained from the ratio B/4TNF (CD19 B lymphocytes/CD4+ T cells producing TNF alpha) derived from quantifying the amount of CD19 B lymphocytes and CD4+ T cells producing TNF alpha in one or more isolated peripheral blood samples obtained from the patient, and
b) comparing the value of said assessment score of step a) with a reference value or reference score,
wherein said corresponding reference (value or score) is linked to a treatment outcome in patients having been treated with alemtuzumab, and
wherein the deviation between the assessment score of step a) with the score or value of reference derived from the comparison step b) is used to predict a treatment outcome for said patient with Multiple Sclerosis when treated with alemtuzumab.

7. The method of claim 6, wherein the amount of CD19 B lymphocytes used to calculate the ratio are selected from the group consisting of the total amount of CD19 B lymphocytes or of the amount of the naive and plasmablast subsets.

8. The method of any of claims 6 or 7, wherein the reference value or score is a cutoff as determined by a receiver operating characteristic (ROC) curve; wherein the ROC curve is based on the distribution of patients having Autoimmune adverse events (AIAEs) in response to treatment with alemtuzumab and patients not having Autoimmune adverse events (AIAEs) in response to treatment with alemtuzumab.

9. The method of claim 8, wherein the cut-off value is of about 0.50.

10. A composition comprising alemtuzumab for use in treating Multiple Sclerosis in a group of patients **characterized by** having a ratio B/4TNF (CD19 B lymphocytes/CD4+ T cells producing TNF alpha) in one or more isolated peripheral blood samples obtained from the patient lower than a reference level, wherein the reference level is a cutoff as determined by a receiver operating characteristic (ROC) curve; wherein the ROC curve is based on the distribution of patients having Autoimmune adverse events (AIAEs) in response to treatment with alemtuzumab and patients not having Autoimmune adverse events (AIAEs) in response to treatment with alemtuzumab.

11. A composition comprising alemtuzumab for use in treating Multiple Sclerosis in a group of patients identified in accordance with the method of any of claims 1 to 5 as having a low risk of autoimmunity if treated with alemtuzumab.

12. A kit comprising antibodies for identifying the ratio B/4TNF (CD19 B lymphocytes/CD4+ T cells producing TNF alpha).

13. The kit of claim 12, wherein the kit which comprises antibodies used to identify the ratio B/4TNF (CD19 B lymphocytes/CD4+ T cells producing TNF alpha) further comprises: (i) a tube or recipient comprising monoclonal antibodies use to identify the ratio B/4TNF (CD19 B lymphocytes/CD4+ T cells producing TNF alpha), or several tubes or recipients comprising separately or in combination each of these antibodies, and (ii) optionally a washing or buffer solution.

14. The kit according to any of claims 12 or 13, to implement any of the methods of claims 1 to 9.

15. Use of the kit according to claim 14, wherein the methods are computer implemented.
